# FASCICULE DE BREVET EUROPEEN

(11) **EP 1 797 028 B1**
(45) Date de publication et mention de la délivrance du brevet: **15.02.2012**
(21) Numéro de dépôt: 05809249.5
(22) Date de dépôt: 04.10.2005
(51) Int. Cl.: C07C 67/307, C07C 69/63, C07B 53/00

(54) **PROCEDE DE PREPARATION STEREOSELECTIF D'UNE MOLECULE FLUOREE CHIRALE**
STEREOSELEKTIVES VERFAHREN ZUR HERSTELLUNG EINES CHIRALEN, FLUORIERTEN MOLEKÜLS
STEREOSELECTIVE METHOD FOR PREPARING A CHIRAL FLUORINATED MOLECULE

(30) Priorité: 04.10.2004 FR 0410450
(43) Date de publication de la demande: 20.06.2007
(73) Titulaire: RHODIA CHIMIE, 93300 Aubervilliers (FR)
(72) Inventeur: LOPEZ, Joseph, F-69007 Lyon (FR); RAJOHARISON, Gérard, F-69390 Vernaison (FR); FERLUT, Jean-Serge, F-69008 Lyon (FR)
(74) Mandataire: Colombet, Alain André
(86) Numéro de dépôt international: PCT/FR2005/002434
(87) Numéro de publication internationale: WO 2006/037887

(56) Documents cités:
- DE-A1- 4 131 242
- US-A- 3 100 225
- E.S.LEWIS, C.E.BOOZER: "The Kinetics and Stereochemistry of the Decomposition of Secondary Alkyl Chlorosulfites" J.AM.CHEM.SOC., vol. 74, 1952, pages 308-311, XP002370709

## Description

La présente invention concerne un procédé de préparation de molécules fluorées chirales, notamment des molécules fluorées possédant un atome de fluor porté par un carbone asymétrique de configuration (R) ou (S), situé en α (alpha) d'un groupe ester ou cétone. Elle concerne en particulier la préparation du (R)-2-fluoropropionate de méthyle (R2F).

Ces composés sont des produits intéressants industriellement notamment comme intermédiaires pour la synthèse d'agents de protection des plantes ou d'insecticides.

Le brevet US 3 100 225 décrit un procédé de production de composés organiques contenant du fluor, par décomposition thermique du composé fluorosulfite correspondant en présence d'une amine tertiaire. Ce document n'enseigne pas un procédé stéréosélectif.

Le brevet DE 4131242 décrit une voie de synthèse stéréosélective du R2F, qui fait intervenir les deux étapes suivantes :
- étape de sulfométhylation 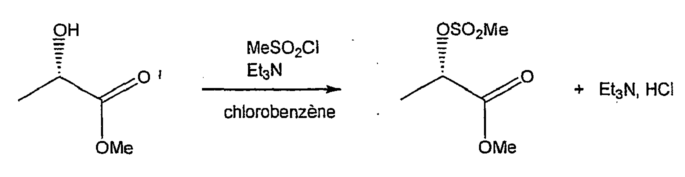
- étape d'échange par KF :

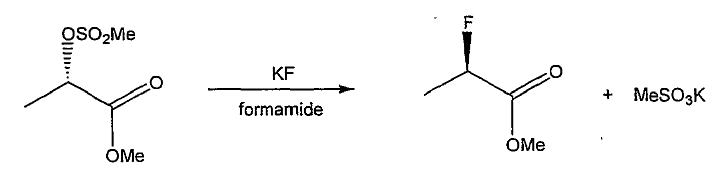

Cette voie d'accès au R2F fonctionne bien d'un point de vue chimique mais présente un inconvénient majeur : génération de quantités d'effluents importantes avec un coût de retraitement très élevé

Les auteurs de la présente invention se sont donc assignés pour objectif de mettre au point un nouveau procédé d'accès stéréosélectif à ces molécules fluorées, d'un rendement satisfaisant à partir de réactifs peu chers et peu générateur d'effluents.

Les auteurs ont réussi à mettre au point un procédé qui remplit cet objectif et qui permet de conduire à des produits optiquement actifs, de configuration déterminée, ayant notamment une pureté optique égale ou supérieure à 95 %

L'invention a ainsi pour objet un procédé de préparation stéréosélectif d'une molécule fluorée chirale, dans lequel :
(i) on introduit dans un réacteur une molécule comprenant un motif C*-OSOF (ci-après dénommé composé fluorosulfite) ;
(2i) on réalise une décomposition thermique de cette molécule, en présence d'un catalyseur nucléophile ;
(3i) on récupère la molécule fluorée produite, comprenant un motif C*-F de configuration inverse par rapport au motif C*-OSOF de départ.

Par nucléophile on entend un catalyseur présentant un atome susceptible de donner un doublet électronique. Conviennent bien les composés comprenant un atome d'azote tertiaire, les sources d'anion fluorure, et les mélanges ou complexes de ceux-ci.

Le catalyseur peut être une amine tertiaire, e.g. le catalyseur peut être choisi parmi : la triéthylamine, la diisopropyléthylamine, la tri-*n*-propylamine, la tri-*n-*butylamine, la méthyldibutylamine, la méthyldicyclohexylamine, l'éthyldiisopropylamine, la N,N-diéthylcyclohexylamine, la pyridine, la diméthylamino-4 pyridine, la N-méthylpipéridine, la N-éthylpipéridine, la N-*n-*butylpipéridine, la 1,2-diméthylpipéridine, la N-méthylpyrrolidine, la 1,2-diméthylpyrrolidine, la diméthylaniline, la picoline, et leurs mélanges.

On peut également utiliser des amides ou des formamides comprenant un azote tertiaire, comme par exemple le diméthylformamide, le diméthylacétamide.

Peuvent aussi être utilisés les dérivés de l'urée tels que les urées substituées par des groupes alkyles, par exemple la tétraméthylurée.

Comme source d'anion fluorure, on peut citer les fluorures basiques tels que KF, les fluorures d'ammonium quaternaire, par exemple fluorure de tétrabutyl ammonium, les fluorures de phosphonium, par exemple fluorure de tétrabutyl phosphonium, et leurs mélanges.

Peuvent aussi être employés les complexes de type HF/amine tertiaire, tels que pyridine/(HF)ₙ ou Et₃N/(HF)ₙ, n étant compris entre 1 et 10.

Dans un mode de réalisation préféré, le catalyseur est la pyridine.

Notamment, on réalise la réaction suivante (I) → (II) : 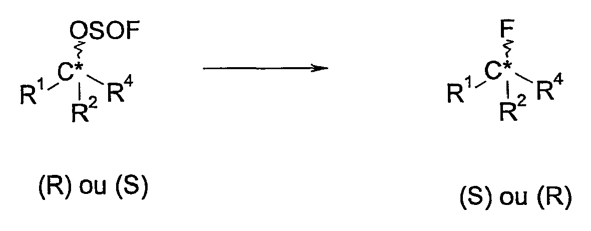 étant précisé que, dans les formules ci-dessus,
- R¹, R² et R⁴ représentent chacun soit un atome d'hydrogène, soit un groupe alkyle, alcényle, alcynyle, ces groupes pouvant être linéaires ou ramifiés, un groupe aryle, cycloalkyle, alkylcycloalkyle, -CO₂R⁵, -(CH₂)ₙ-CO₂R⁵, -COR⁵, -SOR⁵, -SO₂R⁵, n étant un nombre entier de préférence compris entre 1 et 12,
   R⁵ étant l'hydrogène ou un groupe alkyle, alcényle, alcynyle, ces groupes pouvant être linéaires ou ramifiés, cycloalkyle, alkylcycloalkyle, aryle notamment aryle substitué ;
   R¹ pouvant en outre former un hétérocycle aromatique ou non comprenant en remplacement d'un ou plusieurs atomes de carbone un ou plusieurs hétéroatomes choisis parmi l'oxygène, le soufre ou l'azote ;
- R¹, R² et R⁴ étant tous différents.

On réalise soit la réaction suivante (la) → (IIa) : 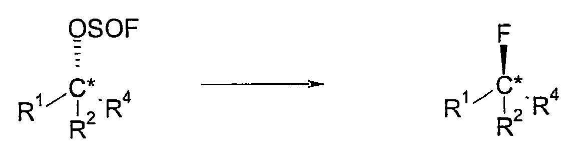 soit la réaction suivante (Ib) → (IIb) :

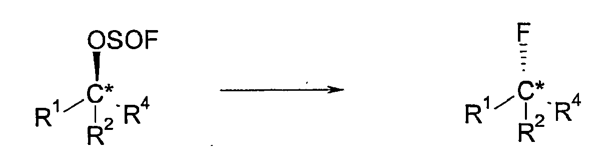

Suivant un mode de réalisation préféré, l'invention a pour objet un procédé de préparation d'une molécule fluorée, possédant un atome de fluor porté par un carbone asymétrique de configuration donnée, situé en α d'un groupe cétone ou ester, procédé dans lequel :
(i) on introduit dans un réacteur un composé comprenant un groupe fluorosulfite de configuration donnée sur le C* portant le groupe fluorosulfite, de formule (III) 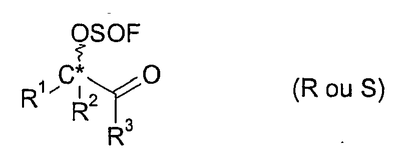
(2i) on réalise la décomposition thermique du composé fluorosulfite en présence d'un catalyseur nucléophile, de préférence un catalyseur comprenant un atome d'azote tertiaire,
(3i) on récupère la molécule fluorée produite, de configuration inverse, de formule (IV) 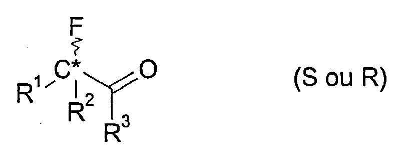 étant précisé que :
   - R¹ représente un groupe alkyle, alcényle, alcynyle, ces groupes pouvant être linéaires ou ramifiés, un groupe aryle, cycloalkyle, alkylcycloalkyle, -CO₂R⁵, -(CH₂)ₙ-CO₂R⁵, -COR⁵, -SOR⁵, -SO₂R⁵,
      n étant un nombre entier compris de préférence entre 1 et 12 R⁵ étant l'hydrogène ou un groupe alkyle, alcényle, alcynyle, ces groupes pouvant être linéaires ou ramifiés, cycloalkyle, alkylcycloalkyle, aryle notamment aryle substitué ;
      R¹ pouvant en outre former un hétérocycle aromatique ou non comprenant en remplacement d'un ou plusieurs atomes de carbone un ou plusieurs hétéroatomes choisis parmi l'oxygène, le soufre ou l'azote ;
   - R² représente l'hydrogène ou un groupe répondant à la définition donnée pour R¹ ;
   - R¹ et R² sont différents ;
   - R³ représente l'hydrogène ou un groupe R⁶ ou -OR⁶, avec un R⁶ choisi dans la liste donnée pour R⁵ ; R⁶ et R¹ pouvant être identiques ou différents.

Dans l'invention, les groupes alkyle, alcényle et alcynyle peuvent comprendre de 1 à 12 atomes de carbone, de préférence de 1 à 6 atomes de carbone. Les groupes aryle, cycloalkyle, alkylcycloalkyle peuvent comprendre de 3 à 8 atomes de carbone, de préférence de 5 à 6 atomes de carbone. Les hétérocycles peuvent comprendre de 3 à 8 atomes dans le cycle, de préférence de 5 à 6.
R² peut notamment représenter l'hydrogène.
R³ peut notamment représenter -OR⁶.
R¹ peut notamment être un groupe alkyle en C₁-C₁₂, de préférence en C₁-C₆, e.g. méthyle.
R⁶ peut notamment être un groupe alkyle en C₁-C₁₂, de préférence en C₁-C₆, e.g. méthyle.

Suivant un aspect particulier de l'invention, le procédé est appliqué aux composés de formule (III), de type lactate, dans lesquels R¹ est méthyle, R² est l'hydrogène et R³ est -Oalkyle.

Suivant une modalité particulière de l'invention, R¹ est méthyle, R² est l'hydrogène et R³ est -OMe, et la configuration de la molécule fluorée est la configuration (R).

De manière particulièrement préférée, la masse de composé fluorosulfite (I), de préférence de formule (III), engagée est substantiellement ou totalement dépourvue de HF et HCl.

Dans les conditions de l'invention, la décomposition du composé fluorosulfite se fait avec inversion de configuration sur le carbone asymétrique (réaction stéréosélective).

La décomposition peut être conduite soit en augmentant progressivement la température du milieu, soit en opérant à une température fixée.

Ainsi, le catalyseur peut être introduit dans le composé fluorosulfite (I), de préférence de formule (III), puis la température peut être augmentée à une valeur suffisante pour initier la décomposition, e.g. entre 60 et 180° C, de préférence entre 100 et 150° C. Le catalyseur est donc ajouté dans le composé fluorosulfite qui se trouve à une température inférieure à la température de décomposition conduisant à l'élimination de SO₂. Le composé fluorosulfite peut ainsi être mis en oeuvre par exemple à la température ambiante (environ 20-25 °C). Un solvant peut être employé lors de cette réaction, le composé fluorosulfite étant introduit dans le solvant, ainsi que le catalyseur, puis la température est augmentée (définition du solvant donnée plus loin).

Dans un procédé à température fixe, on ajoute progressivement le composé fluorosulfite (I), de préférence de formule (III), à décomposer, dans un pied de réaction porté et maintenu à une température adaptée à la décomposition, e.g. comprise entre 60 et 180 °C, de préférence entre 100 et 150 °C, le catalyseur étant présent dans le pied ou ajouté avec ou après le fluorosulfite. Ce pied peut comprendre un solvant ou être formé à partir d'une partie du composé fluorosulfite, de préférence de formule (III), ou de la masse réactionnelle ayant produit le fluorosulfite dans une étape précédente. Ce mode de réalisation permet de conduire cette étape en continu, en ajustant l'admission de composé fluorosulfite à décomposer et le soutirage de la masse réactionnelle.

Comme solvant utilisable dans ces deux modes de réalisation, on peut citer :
- les hydrocarbures aliphatiques et plus particulièrement les paraffines tels que notamment, le pentane, l'hexane, l'heptane, l'octane, l'isooctane, le nonane, le décane, l'undécane, le tétradécane, l'éther de pétrole et le cyclohexane ; les hydrocarbures aromatiques comme notamment le benzène, le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso®.
- les hydrocarbures halogénés aliphatiques ou aromatiques, et l'on peut mentionner : le difluorobenzène, le trifluorométhylbenzène, le fluorobenzène, le monochlorobenzène, le 1,2-dichlorobenzène, le 1,3-dichlorobenzène, le 1,4-dichlorobenzène ou des mélanges,

- les éther-oxydes aliphatiques, cycloaliphatiques ou aromatiques et, plus particulièrement, le méthyl-tert-butyléther, l'oxyde de dipentyle, l'oxyde de diisopentyle, le diméthyléther de l'éthylèneglycol (ou 1,2-diméthoxyéthane), le diméthyléther du diéthylèneglycol (ou 1,5-diméthoxy-3-oxapentane) ou les éthers cycliques, par exemple, le dioxane, le tétrahydrofurane,
- les nitriles aliphatiques ou aromatiques comme l'acétonitrile, le propionitrile, le butanenitrile, l'isobutanenitrile, le benzonitrile, le cyanure de benzyle,
- la N-méthylpyrrolidone.

Dans un procédé continu, le mode de réalisation dans lequel le composé fluorosulfite est alimenté dans un milieu maintenu à la température souhaitée est le mode préféré.

La quantité de catalyseur mise en oeuvre est avantageusement comprise entre 0,1 et 10 % molaire par rapport au composé fluorosulfite, de préférence entre 0,1 et 2 % molaire. On opère de préférence sous une pression comprise entre 50 mbar et 10 bar, plus préférentiellement entre 1 et 10 bars.

Lorsque la décomposition est achevée (il faut typiquement compter de plusieurs dizaines de minutes à plusieurs heures, e.g. de 1 à 5 heures), le milieu peut être refroidi. On peut ensuite procéder à un ou plusieurs lavages à l'eau, puis purifier le brut lavé, par exemple, par distillation sous vide, avant de récupérer le produit pur.

Le composé fluorosulfite (III) peut être obtenu en faisant réagir HF sur le composé chlorosulfite (par définition, composé comprenant un groupe chlorosulfite) correspondant de formule (V), comprenant un groupement OSOCI à la place du groupement OSOF à la formule (III) :

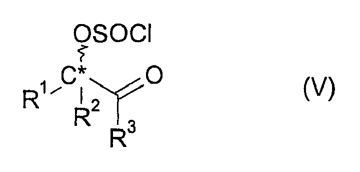

R¹, R² et R³ ayant les mêmes significations que pour les formules (III) et (IV).

La réaction est menée en milieu HF liquide.

Suivant ce mode de mise en oeuvre, on engage généralement de 1 à 10 équivalents de HF par rapport au composé chlorosulfite, de préférence de 1 à 5 équivalents. On préfère ajouter HF sur le composé chlorosulfite. On préfère également opérer sous atmosphère inerte, de préférence azote. La pression absolue est avantageusement suffisante pour maintenir HF à l'état liquide dans les conditions de température. Cette pression peut être par exemple comprise entre la pression atmosphérique et 10 bars. On opère ainsi avantageusement à une température comprise entre - 30 et + 50 °C, de préférence entre - 10 et + 20 °C.

Après introduction de l'HF liquide, le milieu est avantageusement laissé sous agitation à la température souhaitée, pendant un temps suffisant pour conduire la réaction à son terme, ce temps pouvant varier typiquement de 1 à 10 heures selon la température de réaction.

En vue de l'étape de décomposition subséquente, il est préférable d'éliminer HF résiduel et HCl formé. Ceci peut être obtenu, par exemple, en opérant sous balayage d'azote (ou autre gaz inerte) en cours de réaction. HF et HCl sont de préférence éliminés en fin de réaction, par exemple par balayage à l'aide d'un gaz inerte (e.g. azote), combiné de préférence à un chauffage du milieu à une température favorisant l'élimination de HF et HCl dissous (température par exemple comprise entre 20 et 80 °C, e.g. de l'ordre de 50 °C), pendant plusieurs heures. HF et HCl peuvent également être éliminés sous pression réduite L'emploi d'un solvant (e.g. tel que décrit supra) lors de cette étape n'est pas exclu, étant entendu que l'on préfère opérer sans solvant.

Le composé chlorosulfite peut être obtenu en faisant réagir SOCl₂ sur le précurseur hydroxylé (VI) correspondant, comprenant un groupement OH à la place du groupement OSOCI du composé chlorosulfite :

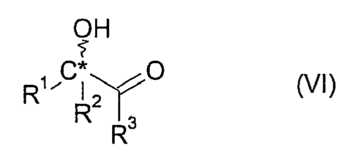

R¹, R² et R³ ayant les mêmes significations que pour les formules (III) et (IV).

On opère avantageusement avec une quantité de SOCl₂ comprise entre 1 et 10 équivalents de SOCl₂ par rapport au précurseur hydroxylé, de préférence entre 1 et 2 équivalents. La température est avantageusement comprise entre - 30 et + 50 °C, de préférence entre - 10 et + 20 °C. En pratique, on préfère couler progressivement (typiquement en 1 à 10h) le précurseur sur un pied de SOCl₂. On préfère aussi opérer sous balayage d'azote. Le pied de chlorure de thionyle est de préférence agité lors de l'ajout du précurseur, puis cette agitation est avantageusement maintenue pendant la durée de finition (typiquement de 1 à 10 heures).

L'emploi d'un solvant (e.g. tel que décrit supra) lors de cette étape n'est pas exclu, étant entendu que l'on préfère opérer sans solvant.

Ces procédés de production du fluorosulfite d'une part et du chlorosulfite d'autre part peuvent être employés dans le but d'obtenir l'ensemble des composés fluorosulfites de formule (I), en partant du chlorosulfite ou du précurseur hydroxylé correspondant au composé de formule (I) retenu.

Suivant un mode de réalisation particulier, on réalise l'enchaînement de réactions suivant, permettant de produire le (R)-2-fluoropropionate de méthyle :

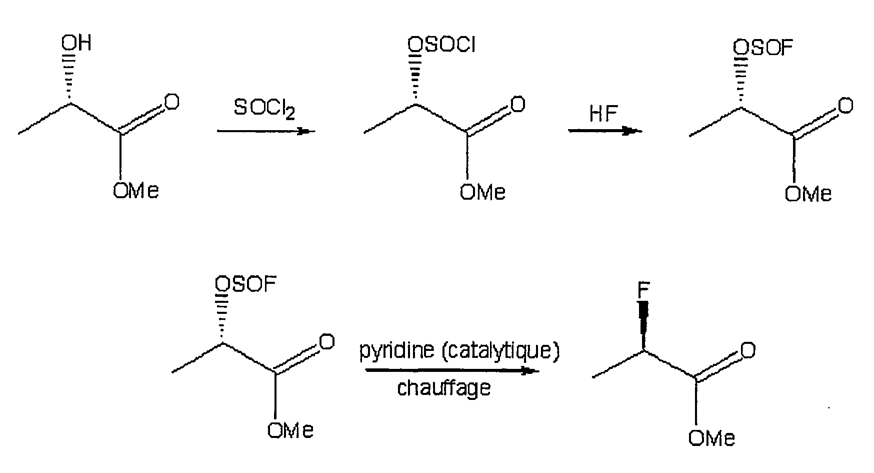

L'enchaînement de réactions peut être réalisé dans le même réacteur ou dans des réacteurs différents.

Différentes voies d'accès aux composés fluorosulfite et en particulier aux composés fluorosulfite (III) peuvent être envisagées. Parmi elles, on peut mentionner la voie comprenant les étapes suivantes :
(i) sur un composé de formule (VI) tel que défini supra faire réagir un composé de formule (VII) SOX₂, les X représentant des atomes d'halogène identiques ou différents, de préférence choisis parmi CI, Br et F, pour obtenir un composé halogénosulfite de formule (VIII) de même configuration 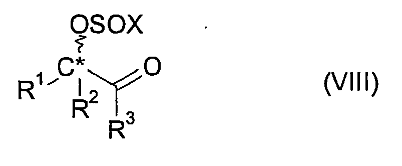
(2i) lorsque un ou les X sont différents de F, faire réagir le composé (VIII) avec HF pour obtenir le composé fluorosulfite de formule (III).

Lorsque les X sont Cl dans la formule (VII), on reconnaît les étapes successives précurseur hydroxylé -> composé chlorosulfite -> composé fluorosulfite décrites en détail supra.

Lorsque les X sont F dans la formule (VII), le composé fluorosulfite est obtenu en une seule étape à partir du précurseur hydroxylé (VI).

Avec SOFCI, on obtient essentiellement et directement le composé fluorosulfite.

Dans le mode de réalisation d'obtention de composé fluorosulfite (III) faisant intervenir SOX₂ avec X représentant un halogène autre que F ou CI, on peut préciser que l'on emploie les mêmes conditions opératoires que par la voie utilisant SOCl₂, puis HF.

L'invention va être maintenant décrite plus en détails à l'aide de la description de modes de réalisation pris à titre d'exemples non limitatifs.

### Etape 1 : Formation du composé chlorosulfite

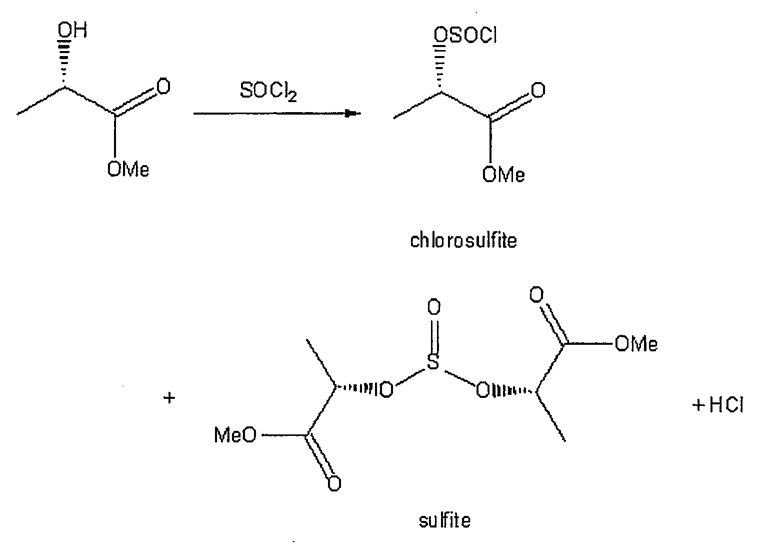

100 g de SOCl₂ (2 équivalents) sont placés en pied de réacteur à 20°C. 43,8 g de (S)-lactate de méthyle sont coulés en 1 heure sous agitation et sous balayage d'azote. L'HCl dégazé est piégé dans une solution aqueuse de soude.

6 h après la fin de la coulée le milieu présente la composition molaire suivante déterminée par RMN (le SOCl₂ résiduel n'a pas été dosé) :
- lactate de méthyle résiduel : 0,1 % (TT=99,9 %)
- chlorosulfite :89,5 % (rendement=80,9%)
- sulfite :10,5 %

### Etape 2 : obtention du composé fluorosulfite

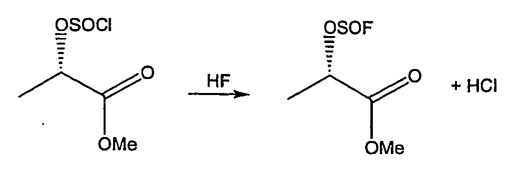

### Etape 3 : décomposition du composé fluorosulfite

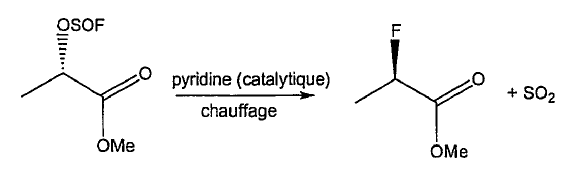

Les étapes 2 et 3 sont enchaînées à partir de la solution de composé chlorosulfite préparée à l'étape 1

La fluoration est réalisée à 10°C en 6h avec 1,5 équivalents d'HF par rapport au composé chlorosulfite introduit. Après stripping à 50°C pendant 15 h sous balayage d'azote, une quantité de pyridine représentant 1,5% molaire par rapport au chlorosulfite initial est introduite. La température du réacteur est ensuite portée à 140°C et maintenue à ce niveau pendant 3 h. Pendant la durée de la décomposition, la pression dans le réacteur est régulée à 2 bar. Le milieu est ensuite refroidi, du dichlorométhane est ajouté puis 2 lavages à l'eau sont réalisés. L'analyse quantitative et l'analyse chirale est faite par chromatographie en phase gazeuse.

Dans ces conditions le rendement en fluoropropionate de méthyle est de 47 % par rapport au composé chlorosulfite engagé.

La pureté optique en énantiomère (R) est de 96,3 %.

## Revendications

1. Procédé de préparation stéréosélectif d'une molécule fluorée chirale, dans lequel :
(i) on introduit dans un réacteur un composé fluorosulfite comprenant un motif C*-OSOF ;
(2i) on réalise une décomposition thermique de cette molécule, en présence d'un catalyseur nucléophile ;
(3i) on récupère la molécule fluorée produite, comprenant un motif C*-F de configuration inverse par rapport au motif C*-OSOF de départ.

2. Procédé selon la revendication 1, dans lequel on réalise la réaction suivante : 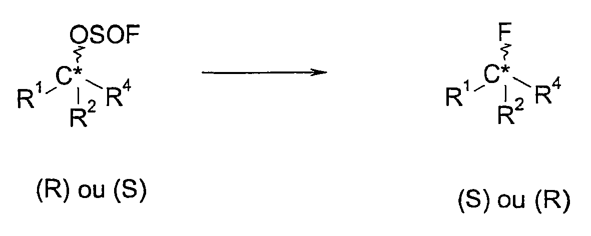 étant précisé que, dans les formules ci-dessus,
- R¹, R² et R⁴ représentent chacun soit un atome d'hydrogène, soit un groupe alkyle, alcényle, alcynyle, ces groupes pouvant être linéaires ou ramifiés, un groupe aryle, cycloalkyle, alkylcycloalkyle, -CO₂R⁵, -(CH₂)ₙ-CO₂R⁵, -COR⁵, -SOR⁵, -SO₂R⁵, n étant un nombre entier de préférence compris entre 1 et 12,
R⁵ étant l'hydrogène ou un groupe alkyle, alcényle, alcynyle, ces groupes pouvant être linéaires ou ramifiés, cycloalkyle, alkylcycloalkyle, aryle notamment aryle substitué ;
R¹ pouvant en outre former un hétérocycle aromatique ou non comprenant en remplacement d'un ou plusieurs atomes de carbone un ou plusieurs hétéroatomes choisis parmi l'oxygène, le soufre ou l'azote ;
- R¹, R² et R⁴ étant tous différents.

3. Procédé selon la revendication 2, dans lequel on réalise la réaction suivante :

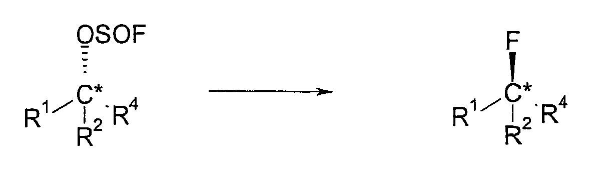

4. Procédé selon la revendication 2, dans lequel on réalise la réaction suivante :

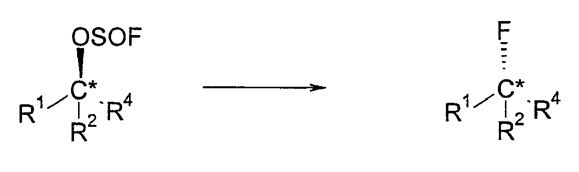

5. Procédé selon l'une des revendications précédentes, pour la préparation d'une molécule fluorée, possédant un atome de fluor porté par un carbone asymétrique de configuration (R) ou (S), situé en α d'un groupe ester ou cétone, procédé dans lequel :
(i) on introduit dans un réacteur un composé fluorosulfite de configuration donnée sur le C* portant le groupe fluorosulfite, de formule (III) 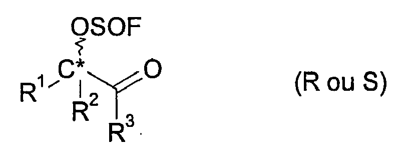
(2i) on réalise la décomposition thermique du composé fluorosulfite en présence d'un catalyseur nucléophile,
(3i) on récupère la molécule fluorée produite, de configuration inverse, de formule (IV) 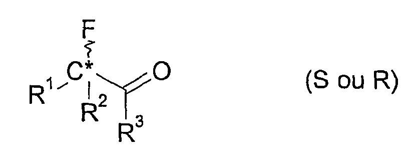 étant précisé que :
- R¹ représente un groupe alkyle, alcényle, alcynyle, ces groupes pouvant être linéaires ou ramifiés, un groupe aryle, cycloalkyle, alkylcycloalkyle, -CO₂R⁵, -(CH₂)ₙ-CO₂R⁵, -COR⁵, -SOR⁵, -SO₂R⁵, n étant un nombre entier de préférence compris entre 1 et 12,
R⁵ étant l'hydrogène ou un groupe alkyle, alcényle, alcynyle, ces groupes pouvant être linéaires ou ramifiés, cycloalkyle, alkylcycloalkyle, aryle notamment aryle substitué ;
R¹ pouvant en outre former un hétérocycle aromatique ou non comprenant en remplacement d'un ou plusieurs atomes de carbone un ou plusieurs hétéroatomes choisis parmi l'oxygène, le soufre ou l'azote ;
- R² représente l'hydrogène ou un groupe répondant à la définition donnée pour R¹ ;
- R¹ et R² sont différents ;
- R³ représente l'hydrogène ou un groupe R⁶ ou -OR⁶, avec un R⁶ choisi dans la liste donnée pour R⁵, R⁶ et R¹ pouvant être identiques ou différents.

6. Procédé selon la revendication 5, dans lequel R³ représente -OR⁶.

7. Procédé selon la revendication 5 ou 6, dans lequel R⁶ est un groupe alkyle en C₁-C₁₂, de préférence en C₁-C₆, e.g. méthyle.

8. Procédé selon l'une des revendications 5 à 7, dans lequel R¹ est méthyle, R² est l'hydrogène et R³ est -Oalkyle, de préférence -OMe.

9. Procédé selon l'une des revendications précédentes, dans lequel R² représente l'hydrogène.

10. Procédé selon l'une des revendications précédentes, dans lequel R¹ est un groupe alkyle en C₁-C₁₂, de préférence en C₁-C₆, e.g. méthyle.

11. Procédé selon l'une des revendications précédentes, dans lequel le catalyseur est un composé comprenant un atome d'azote tertiaire, une source d'anion fluorure, ou un mélange ou complexe de ceux-ci.

12. Procédé selon la revendication 11, dans lequel le catalyseur est choisi parmi : la triéthylamine, la diisopropyléthylamine, la tri-*n*-propylamine, la tri-*n*-butylamine, la méthyldibutylamine, la méthyldicyclohexylamine, l'éthyldiisopropylamine, la N,N-diéthylcyclohexylamine, la pyridine, la diméthylamino-4 pyridine, la N-méthylpipéridine, la N-éthylpipéridine, la N-n-butylpipéridine, la 1,2-diméthylpipéridine, la N-méthylpyrrolidine, la 1,2-diméthylpyrrolidine, la diméthylaniline, la picoline, et leurs mélanges.

13. Procédé selon la revendication 11, dans lequel le catalyseur est choisi parmi les amides et les formamides comprenant un azote tertiaire, les dérivés de l'urée, les fluorures basiques, les fluorures d'ammonium, les fluorures de phosphonium.

14. Procédé selon la revendication 11, dans lequel le catalyseur est la pyridine.

15. Procédé selon l'une des revendications précédentes, dans lequel la masse de composé fluorosulfite engagée est substantiellement ou totalement dépourvue de HF et HCl.

16. Procédé selon l'une des revendications précédentes, dans lequel le catalyseur est introduit dans le composé fluorosulfite, puis la température est augmentée entre 60 et 180° C, de préférence entre 100 et 150° C.

17. Procédé selon l'une quelconque des revendications 1 à 9, dans lequel on ajoute progressivement le composé fluorosulfite dans un solvant porté à une température comprise entre 60 et 180 °C, de préférence entre 100 et 150 °C, le catalyseur étant présent dans le solvant ou ajouté avec ou après le composé fluorosulfite.

18. Procédé selon l'une des revendications précédentes, dans lequel la quantité de catalyseur mise en oeuvre est comprise entre 0,1 et 10 % molaire par rapport au composé fluorosulfite, de préférence entre 0,1 et 2 % molaire.

19. Procédé selon l'une des revendications précédentes, dans lequel on opère sous une pression comprise entre 50 mbar et 10 bar, de préférence entre 1 et 10 bars.

20. Procédé selon l'une des revendications précédentes, dans lequel le composé fluorosulfite est obtenu en faisant réagir HF sur le composé chlorosulfite correspondant, comprenant un groupement OSOCI à la place du groupement OSOF.

21. Procédé selon la revendication 20, dans lequel on engage de 1 à 10 équivalents de HF par rapport au composé chlorosulfite, de préférence de 1 à 5 équivalents.

22. Procédé selon la revendication 20 ou 21, dans lequel on ajoute HF sur le composé chlorosulfite.

23. Procédé selon l'une des revendications 20 à 22, dans lequel on opère sous atmosphère inerte.

24. Procédé selon l'une des revendications 20 à 23, dans lequel on opère à une température comprise entre - 30 et + 50 °C, de préférence entre - 10 et + 20 °C.

25. Procédé selon l'une des revendications 20 à 24, dans lequel on élimine HF et HCl, en fin de réaction entre HF et le composé chlorosulfite.

26. Procédé selon l'une des revendications 20 à 25, dans lequel le composé chlorosulfite est obtenu en faisant réagir SOCl₂ sur le précurseur hydroxylé correspondant, comprenant un groupement OH à la place du groupement OSOCI du composé chlorosulfite.

27. Procédé selon la revendication 26, dans lequel on opère avec une quantité de SOCl₂ comprise entre 1 et 10 équivalents de SOCl₂ par rapport au précurseur hydroxylé, de préférence entre 1 et 2 équivalents.

28. Procédé selon la revendication 26 ou 27, dans lequel on opère à une température comprise entre - 30 et + 50 °C, de préférence entre - 10 et + 20 °C.

29. Procédé selon l'une des revendications 26 à 28, dans lequel on coule progressivement le précurseur sur un pied de SOCl₂.

30. Procédé selon l'une des revendications 26 à 29, dans lequel on opère sous balayage d'azote.

31. Procédé selon l'une des revendications 26 à 30, dans lequel on réalise l'enchaînement de réactions suivant :

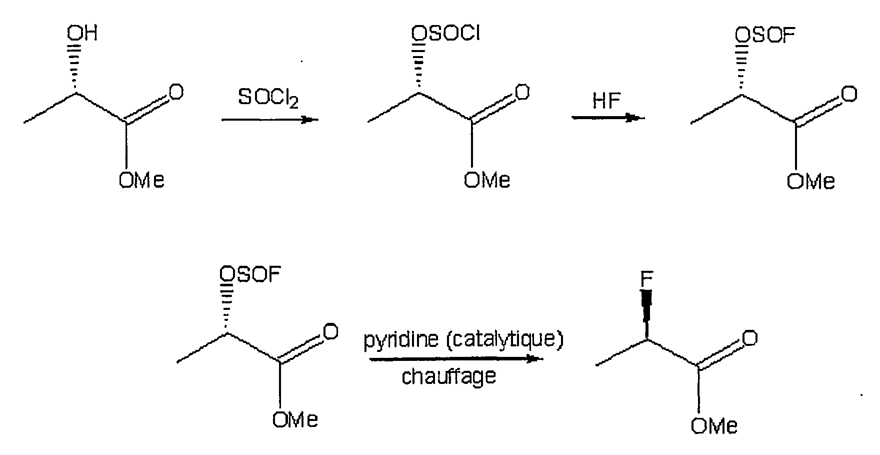

32. Procédé selon la revendication 31, dans lequel cet enchaînement de réactions est réalisé dans le même réacteur ou dans des réacteurs différents.

33. Procédé selon l'une des revendications 5 à 7, dans lequel le composé fluorosulfite (III) est obtenu en faisant réagir SOF₂ sur le précurseur hydroxylé de formule (VI) : 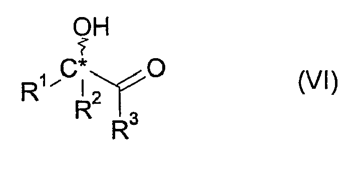 R¹, R² et R³ ayant les mêmes significations que pour les formules (III) et (IV).

## Claims

1. Stereoselective method for preparing a chiral fluorinated molecule, in which:
(i) a fluorosulfite compound containing a C*-OSOF unit is introduced into a reactor;
(2i) thermal decomposition of that molecule is carried out in the presence of a nucleophilic catalyst;
(3i) the resulting fluorinated molecule, containing a C*-F unit having the inverse configuration relative to the starting C*-OSOF unit, is recovered.

2. Method according to claim 1, in which the following reaction is carried out: 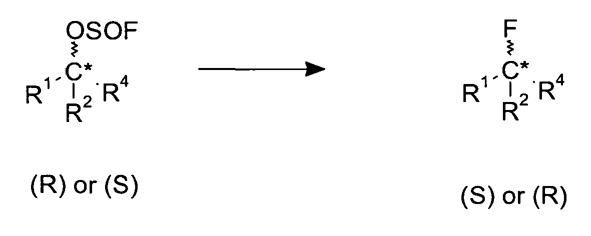 wherein, in the above formulae,
- R¹, R² and R⁴ each represents either a hydrogen atom or a group alkyl, alkenyl, alkynyl, which groups can be linear or branched, a group aryl, cycloalkyl, alkylcycloalkyl, -CO₂R⁵, -(CH₂)ₙ-CO₂R⁵, -COR⁵, -SOR⁵, -SO₂R⁵, n being an integer preferably from 1 to 12,
R⁵ being hydrogen or a group alkyl, alkenyl, alkynyl, which groups can be linear or branched, cycloalkyl, alkylcycloalkyl, aryl, especially substituted aryl;
it further being possible for R¹ to form an aromatic or non-aromatic heterocyclic group containing in place of one or more carbon atoms one or more hetero atoms selected from oxygen, sulfur and nitrogen;
- R¹, R² and R⁴ all being different.

3. Method according to claim 2, in which the following reaction is carried out:

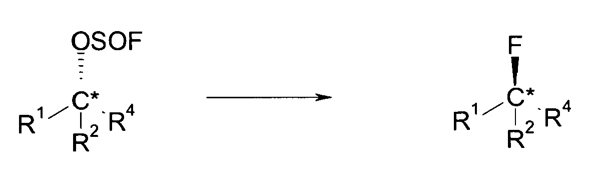

4. Method according to claim 2, in which the following reaction is carried out:

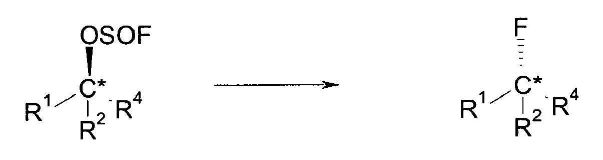

5. Method according to any one of the preceding claims, for preparing a fluorinated molecule having a fluorine atom carried by an asymmetric carbon atom having the (R) or (S) configuration, located α to an ester or ketone group, in which method:
(i) there is introduced into a reactor a fluorosulfite compound having a given configuration at the C* carrying the fluorosulfite group, of formula (III) 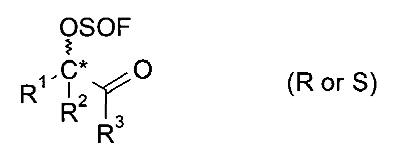
(2i) thermal decomposition of the fluorosulfite compound is carried out in the presence of a nucleophilic catalyst,
(3i) the resulting fluorinated molecule, having the inverse configuration, of formula (IV) is recovered 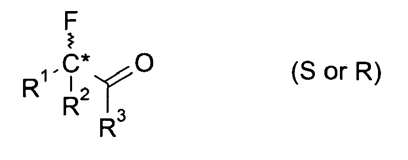
wherein:
- R¹ represents a group alkyl, alkenyl, alkynyl, which groups can be linear or branched, a group aryl, cycloalkyl, alkylcycloalkyl, -CO₂R⁵, -(CH₂)ₙ-CO₂R
- COR⁵, -SOR⁵, -SO₂R⁵,
n being an integer preferably from 1 to 12,
R⁵ being hydrogen or a group alkyl, alkenyl, alkynyl, which groups can be linear or branched, cycloalkyl, alkylcycloalkyl, aryl, especially substituted aryl;
it further being possible for R¹ to form an aromatic or non-aromatic heterocyclic group containing in place of one or more carbon atoms one or more hetero atoms selected from oxygen, sulfur and nitrogen;
- R² represents hydrogen or a group corresponding to the definition given for R¹;
- R¹ and R² are different;
- R³ represents hydrogen or a group R⁶ or -OR⁶, wherein R⁶ is selected from the list given for R⁵ , it being possible for R⁶ and R¹ to be identical or different.

6. Method according to claim 5, in which R³ represents -OR⁶.

7. Method according to claim 5 or 6, in which R⁶ is a C₁-C₁₂-alkyl group, preferably a C₁-C₆-alkyl group, e.g. methyl.

8. Method according to any one of claims 5 to 7, in which R¹ is methyl, R² is hydrogen and R³ is -Oalkyl, preferably -OMe.

9. Method according to any one of the preceding claims, in which R² represents hydrogen.

10. Method according to any one of the preceding claims, in which R¹ is a C₁-C₁₂-alkyl group, preferably a C₁-C₆-alkyl group, e.g. methyl.

11. Method according to any one of the preceding claims, in which the catalyst is a compound containing a tertiary nitrogen atom, a fluoride anion source, or a mixture or complex thereof.

12. Method according to claim 11, in which the catalyst is selected from: triethylamine, diisopropylethylamine, tri-n-propylamine, tri-n-butylamine, methyldibutylamine, methyldicyclohexylamine, ethyldiisopropylamine, N,N-diethylcyclohexylamine, pyridine, 4-dimethylaminopyridine, N-methylpiperidine, N-ethylpiperidine, N-n-butylpiperidine, 1,2-dimethylpiperidine, N-methylpyrrolidine, 1,2-dimethylpyrrolidine, dimethylaniline, picoline, and mixtures thereof.

13. Method according to claim 11, in which the catalyst is selected from amides and formamides containing a tertiary nitrogen atom, urea derivatives, basic fluorides, ammonium fluorides, phosphonium fluorides.

14. Method according to claim 11, in which the catalyst is pyridine.

15. Method according to any one of the preceding claims, in which the mass of fluorosulfite compound used is substantially or totally free of HF and HCl.

16. Method according to any one of the preceding claims, in which the catalyst is introduced into the fluorosulfite compound and then the temperature is increased to 60 to 180°C, preferably 100 to 150°C.

17. Method according to any one of claims 1 to 9, in which the fluorosulfite compound is added gradually to a solvent heated to a temperature of from 60 to 180°C, preferably from 100 to 150°C, the catalyst being present in the solvent or being added with or after the fluorosulfite compound.

18. Method according to any one of the preceding claims, in which the amount of catalyst used is from 0.1 to 10 mol.%, based on the fluorosulfite compound, preferably from 0.1 to 2 mol.%.

19. Method according to any one of the preceding claims, in which the procedure is carried out under a pressure of from 50 mbar to 10 bar, preferably from 1 to 10 bar.

20. Method according to any one of the preceding claims, in which the fluorosulfite compound is obtained by reacting HF with the corresponding chlorosulfite compound containing a OSOCl group instead of the OSOF group.

21. Method according to claim 20, in which there are used from 1 to 10 equivalents of HF, based on the chlorosulfite compound, preferably from 1 to 5 equivalents.

22. Method according to claim 20 or 21, in which HF is added to the chlorosulfite compound.

23. Method according to any one of claims 20 to 22, in which the procedure is carried out under an inert atmosphere.

24. Method according to any one of claims 20 to 23, in which the procedure is carried out at a temperature of from -30 to +50°C, preferably from -10 to +20°C.

25. Method according to any one of claims 20 to 24, in which HF and HCl are removed at the end of the reaction between HF and the chlorosulfite compound.

26. Method according to any one of claims 20 to 25, in which the chlorosulfite compound is obtained by reacting SOCl₂ with the corresponding hydroxylated precursor containing a OH group instead of the OSOCI group of the chlorosulfite compound.

27. Method according to claim 26, in which the procedure is carried out with an amount of SOCl₂ of from 1 to 10 equivalents of SOCl₂, based on the hydroxylated precursor, preferably from 1 to 2 equivalents.

28. Method according to claim 26 or 27, in which the procedure is carried out at a temperature of from -30 to +50°C, preferably from -10 to +20°C.

29. Method according to any one of claims 26 to 28, in which the precursor is poured gradually onto a base of SOCl₂.

30. Method according to any one of claims 26 to 29, in which the procedure is carried out with nitrogen flushing.

31. Method according to any one of claims 26 to 30, in which the following sequence of reactions is carried out:

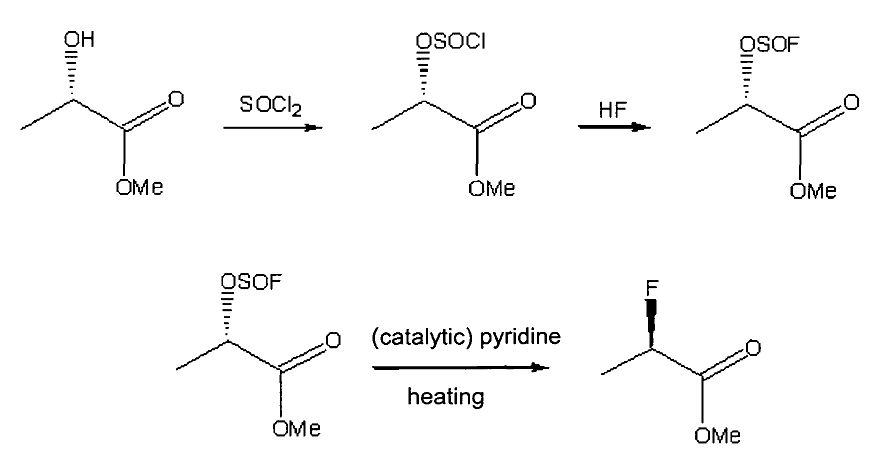

32. Method according to claim 31, in which the sequence of reactions is carried out in the same reactor or in different reactors.

33. Method according to any one of claims 5 to 7, in which the fluorosulfite compound (III) is obtained by reacting SOF₂ with the hydroxylated precursor of formula (VI): 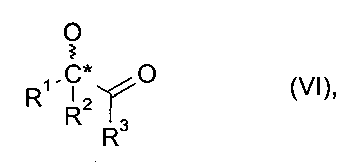 R¹, R² and R³ having the same meanings as for formulae (III) and (IV).

## Patentansprüche

1. Stereoselektives Verfahren zur Herstellung eines chiralen fluorierten Moleküls, bei dem:
(i) in einen Reaktor eine Fluorsulfitverbindung umfassend eine Einheit C*-OSOF eingeführt wird;
(2i) ein thermischer Zerfall dieses Moleküls in Anwesenheit eines nukleophilen Katalysators realisiert wird;
(3i) das erzeugte fluorierte Molekül, umfassend eine Einheit C*-F von in Bezug auf die Ausgangseinheit C*-OSOF inverser Konfiguration zurückgewonnen wird.

2. Verfahren nach Anspruch 1, bei dem die folgende Reaktion realisiert wird: 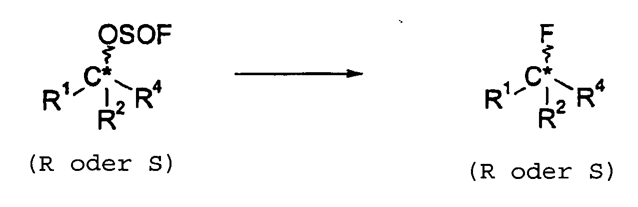 wobei klargestellt wird, dass in den oben genannten Formeln
- R¹, R² und R⁴ jeweils sei es ein Wasserstoffatom, sei es eine Alkyl-, Alkenyl-, Alkinylgruppe, wobei diese Gruppen linear oder verzweigt sein können, eine Aryl-, Cycloalkyl-, Alykylcycloalkylgruppe, - CO₂R⁵, - (CH₂)ₙ-CO₂R⁵, COR⁵, -SOR⁵, -SO₂R⁵, wobei n eine ganze Zahl, vorzugsweise zwischen 1 und 12 ist, darstellen, wobei R⁵ Wasserstoff oder eine Alkyl-, Alkenyl-, Alkinylgruppe, wobei diese Gruppen linear oder verzweigt sein können, Cycloalkyl, Alkylcycloalkyl, Aryl, insbesondere substituiertes Aryl ist;
wobei R¹ außerdem eine aromatische heterocyklische Verbindung bilden kann oder nicht, die in Ersetzung von einem oder mehreren Kohlenstoffatome ein oder mehrere Heteroatome, gewählt zwischen Sauerstoff,
Schwefel oder Stickstoff, umfasst;
- R¹, R² und R⁴ alle unterschiedlich sind.

3. Verfahren nach Anspruch 2, bei dem die folgende Reaktion realisiert wird:

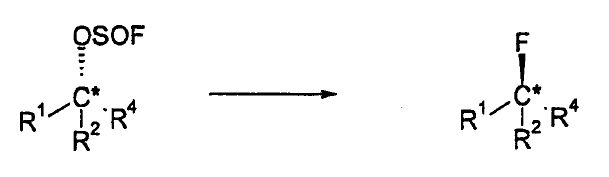

4. Verfahren nach Anspruch 2, bei dem die folgende Reaktion realisiert wird:

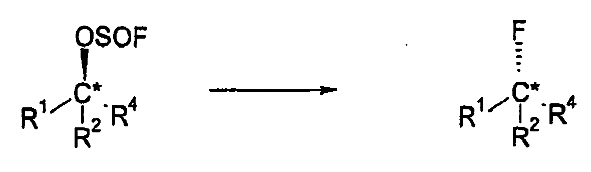

5. Verfahren nach einem der vorhergehenden Ansprüche für die Herstellung eines fluorierten Moleküls, das ein von einem asymmetrischen Kohlenstoff der Konfiguration (R) oder (S) getragenen Fluoratom, angeordnet in α einer Ester- oder Ketongruppe, besitzt, wobei bei dem Verfahren:
(i) in einen Reaktor eine Fluorsulfitverbindung einer auf dem C* gegebenen Konfiguration, die die Fluorsulfitgruppe trägt, der Formel (III) einführt 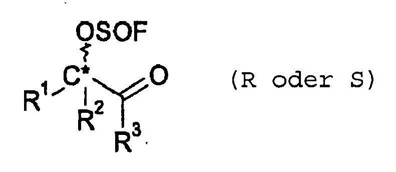
(2i) ein thermischer Zerfall dieser Fluorsulfitverbindung in Anwesenheit eines nukleophilen Katalysators realisiert wird,
(3i) das erzeugte fluorierte Molekül von inverser Konfiguration der Formel (IV) zurückgewonnen wird 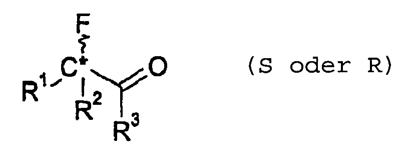
wobei klargestellt wird, dass:
- R¹ eine Alkyl-, Alkenyl-, Alcynylgruppe, wobei diese Gruppen linear oder verzweigt sein können, eine Aryl-, Zykloalkyl-, Alkylzykloalkylgruppe -CO₂R⁵, -(CH₂)ₙ-CO₂R⁵, COR⁵,-SOR⁵, -SO₂R⁵, wobei n eine ganze Zahl, vorzugsweise zwischen 1 und 12 ist, repräsentiert,
wobei R⁵ Wasserstoff oder eine Alkyl-, Alkenyl-, Alcynylgruppe, wobei diese Gruppen linear oder verzweigt sein können, Zykloalkyl, Alkylzykloalkyl, Aryl, insbesondere substituiertes Aryl ist;
wobei R¹ außerdem eine aromatische heterozyklische Verbindung bilden kann oder nicht, die in Ersetzung eines oder mehrerer Kohlenstoffatome ein oder mehrere Heteroatome, gewählt zwischen Sauerstoff, Schwefel oder Stickstoff, umfasst;
- R² Wasserstoff oder eine Gruppe, die der für R¹ gegebenen Definition entspricht, repräsentiert;
- R¹ und R² unterschiedlich sind;
- R³ Wasserstoff oder eine Gruppe R⁶ oder -OR⁶ darstellt, mit R⁶ gewählt aus der Liste, die für R⁵ gegeben ist, wobei R⁶ und R¹ identisch oder unterschiedlich sein können.

6. Verfahren nach Anspruch 5, bei dem R³ -OR⁶ darstellt.

7. Verfahren nach Anspruch 5 oder 6, bei dem R⁶ eine C₁ - C₁₂ Alkylgruppe, vorzugsweise eine C₁ - C₆ Alkylgruppe, z.B. Methyl ist.

8. Verfahren nach einem der Ansprüche 5 bis 7, bei dem R¹ Methyl ist, R² Wasserstoff ist und R³ -Oalkyl, vorzugsweise -OMe ist.

9. Verfahren nach einem der vorhergehenden Ansprüche, bei dem R² Wasserstoff darstellt.

10. Verfahren nach einem der vorhergehenden Ansprüche, bei dem R¹ eine Alkylgruppe in C₁ - C₁₂, vorzugsweise in C₁ - C₆, z.B. Methyl ist.

11. Verfahren nach einem der vorhergehenden Ansprüche, bei dem der Katalysator eine Verbindung ist, die ein tertiäres Stickstoffatom, eine Fluoranionquelle oder eine Mischung oder ein Komplex aus diesen umfasst.

12. Verfahren nach Anspruch 11, bei dem der Katalysator gewählt ist unter: Triethylamin, Diisopropylethylamin, Tri-n-propylamin, Tri-n-Butylamin, Methyldibutylamin, Methyldizyklohexylamin, Ethyldiisopropylamin, N, N-Diethylzyklohexylamin, Pyridin, 4-(Dimethylamino)-pyridin, N-Methylpiperidin, N-Ethylpiperidin, N-n-Butylpiperidin, 1,2-Dimethylpiperidin, N-Methylpyrrolidin, 1,2-Dimethylpyrrolidin, Dimethylanilin, Picolin und ihren Mischungen.

13. Verfahren nach Anspruch 11, bei dem der Katalysator zwischen den Amiden und den Formamiden, die einen tertiären Stickstoff umfassen, den Harnstoffderivaten, den basischen Fluoriden, den Amoniumfluoriden, den Phosphoniumfluoriden gewählt ist.

14. Verfahren nach Anspruch 11, bei dem der Katalysator Pyridin ist.

15. Verfahren nach einem der vorhergehenden Ansprüche, bei dem die eingesetzte Masse der Fluorsulfitverbindung im Wesentlichen oder insgesamt kein HF und HCl enthält.

16. Verfahren nach einem der vorhergehenden Ansprüche, bei dem der Katalysator in die Fluorsulfitverbindung eingebracht wird, dann die Temperatur auf eine solche zwischen 60 und 180°C, vorzugsweise zwischen 100 und 150°C erhöht wird.

17. Verfahren nach einem beliebigen der Ansprüche 1 bis 9, bei dem schrittweise die Fluorsulfitverbindung in eine Lösung gebracht wird, die auf eine Temperatur zwischen 60 und 180°C, vorzugsweise zwischen 100 und 150°C gebracht ist, wobei der Katalysator in der Lösung vorhanden ist oder mit oder nach der Fluorsulfitverbindung hinzugefügt wird.

18. Verfahren nach einem der vorhergehenden Ansprüche, bei dem die umgesetzte Katalysatormenge zwischen 0,1 und 10 Mol% in Bezug auf die Fluorsulfitverbindung, vorzugsweise zwischen 0,1 und 2 Mol% liegt.

19. Verfahren nach einem der vorhergehenden Ansprüche, bei dem bei einem Druck zwischen 50 mbar und 10 bar, vorzugsweise zwischen 1 und 10 bar gearbeitet wird.

20. Verfahren nach einem der vorhergehenden Ansprüche, bei dem die Fluorsulfitverbindung erhalten wird, indem man HF auf die entsprechende Chlorsulfitverbindung reagieren lässt, die eine Gruppierung OSOCl anstelle der Gruppierung OSOF umfasst.

21. Verfahren nach Anspruch 20, bei dem 1 bis 10 Äquivalente des HF in Bezug auf die Chlorsulfitverbindung, vorzugsweise zwischen 1 bis 5 Äquivalente eingesetzt werden.

22. Verfahren nach Anspruch 20 oder 21, bei dem HF der Chlorsulfitverbindung hinzugefügt wird.

23. Verfahren nach einem der Ansprüche 20 bis 22, bei dem bei inerter Atmosphäre gearbeitet wird.

24. Verfahren nach einem der Ansprüche 20 bis 23, bei dem bei einer Temperatur zwischen -30 und 50°C, vorzugsweise zwischen -10 und +20°C gearbeitet wird.

25. Verfahren nach einem der Ansprüche 20 bis 24, bei dem HF und HCl am Ende der Reaktion zwischen HF und der Chlorsulfitverbindung eliminiert werden.

26. Verfahren nach einem der Ansprüche 20 bis 25, bei dem die Chlorsulfitverbindung erhalten wird, indem man SOCl₂ auf den entsprechenden Hydroxylzwischenstoff, der eine Gruppierung OH anstelle der Gruppierung OSOCl der Chlorsulfitverbindung enthält, reagieren lässt.

27. Verfahren nach Anspruch 26, bei dem mit einer SOCl₂ Menge zwischen 1 und 10 Äquivalenten von SOCl₂ in Bezug auf den Hydroxylzwischenstoff, vorzugsweise zwischen 1 und 2 Äquivalenten gearbeitet wird.

28. Verfahren nach Anspruch 26 oder 27, bei dem bei einer Temperatur zwischen -30 und +50°C, vorzugsweise zwischen -10 und +20°C gearbeitet wird.

29. Verfahren nach einem der Ansprüche 26 bis 28, bei dem man schrittweise den Zwischenstoff auf SOCl₂ gießt.

30. Verfahren nach einem der Ansprüche 26 bis 29, bei dem unter Stickstoffspülung gearbeitet wird.

31. Verfahren nach einem der Ansprüche 26 bis 30, bei dem die folgende Reaktionskette hergestellt wird:

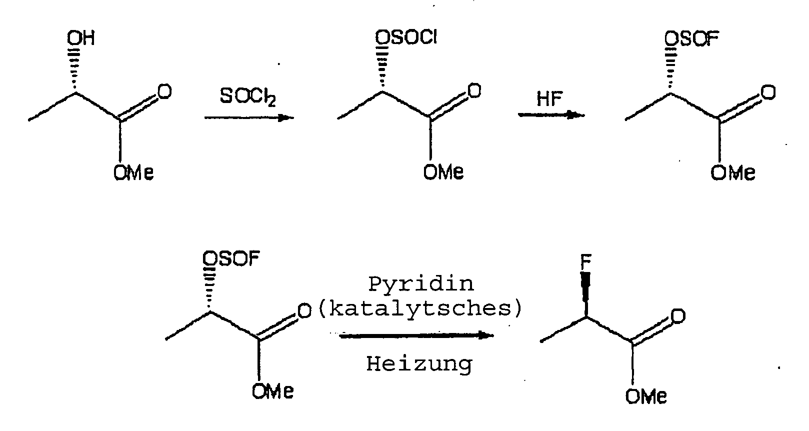

32. Verfahren nach Anspruch 31, bei dem diese Reaktionskette in demselben Reaktor oder in unterschiedlichen Reaktoren realisiert wird.

33. Verfahren nach einem der Ansprüche 5 bis 7, bei dem die Fluorsulfitverbindung (III) erhalten wird, indem man SOF₂ auf den Hydroxylzwischenstoff der Formel (VI) reagieren lässt: 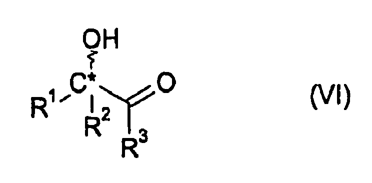 wobei R¹, R² und R³ dieselben Bedeutungen wie für die Formeln (III) und (IV) haben.
